Europäisches Patentamt

European Patent Office

Office européen des brevets

(11) Publication number: **0 243 058 B1**

# EUROPEAN PATENT SPECIFICATION

(45) Date of publication of patent specification: **28.07.93**

(51) Int. Cl.⁵: **C07K 15/06**, C12P 21/00, C12N 5/00, A61K 39/395, A61K 43/00, A61K 49/02, G01N 33/574, G01N 33/577, //C12N15/00,(C12P21/00, C12R1:91)

(21) Application number: **87303194.2**

(22) Date of filing: **13.04.87**

(54) A tumor associated antigen.

(30) Priority: **17.04.86 US 853067**

(43) Date of publication of application:
**28.10.87 Bulletin 87/44**

(45) Publication of the grant of the patent:
**28.07.93 Bulletin 93/30**

(84) Designated Contracting States:
**AT BE CH DE ES FR GB GR IT LI LU NL SE**

(56) References cited:
**EP-A- 0 242 154**
**WO-A-85/03132**
**DE-A- 3 329 184**

**CHEMICAL ABSTRACTS, vol. 104, no. 17, 28th April 1986, page 503, abstract no. 146685q, Columbus, Ohio, US; A.M. CARROLL et al.: "Biochemical characterization of a prostate tissue antigen"**

(73) Proprietor: **HYBRITECH INCORPORATED**
**11095 Torreyana Road**
**San Diego California 92126(US)**

(72) Inventor: **Burnett, Karen Gray**
**10295 Rue Chamberry**
**San Diego, CA 92131(US)**
Inventor: **Grauer, Lana Suzanne**
**13252 Caminito Point Del Mar**
**Del Mar, CA 92014(US)**
Inventor: **Oh, Esther Hui-Yim**
**11117 Pegasus Avenue**
**San Diego, CA 92126(US)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

FEDERATION PROCEEDINGS, vol. 45, no. 4, 1986, page 984, abstract no. 4830; E. OH et al.: "A breast cancer antigen targeted in vivo by a human monoclonal antibody"

PROCEEDINGS OF THE NATIONAL ACADEMY OF SCIENCES, USA, vol. 79, September 1982, pages 5420-5424; R.L. CERIANI et al.: "Circulating human mammary epithelial antigens in breast cancer"

PROCEEDINGS OF THE NATIONAL ACADEMY OF SCIENCES, USA, vol. 77, no. 11, November 1980, pages 6841-6845; J. SCHLOM et al.: "Generation of human monoclonal antibodies reactive with human mammary carcinoma cells"

## Description

The present invention relates to the characterization of antigens, particularly tumor-associated antigens. In another aspect, it relates to antibodies having specific reactivity with such antigens. In yet another aspect, it relates to methods for producing such antibodies as well as diagnostic and therapeutic uses therefor.

The potential role of monoclonal antibodies in the diagnosis and treatment of cancer in humans has been the focus of much recent investigation and speculation. Of particular interest are their use in immunoassays to monitor the course of disease, e.g., during therapy. Also of particular interest are the potential applications of monoclonal antibodies for tumor imaging and therapy due to their capacity to seek out and selectively bind to tumor-associated antigens in vivo.

Developments in monoclonal antibody technology have also made it possible to investigate the antigenic complexity of human tumors. Specifically, the precise immunoreactivity of monoclonal antibodies permits the identification and differentiation of antigens expressed by human tumors. The characterization of such distinct tumor-associated antigens, therefore, provides a means to more fully exploit the use of monoclonal antibodies for cancer diagnosis and therapy and to maximize the advance of monoclonal antibody technology.

Paradoxically, these distinct antigens are expressed not only by human tumor cells but also by normal cells of the same type. Accordingly, these antigens are not tumor specific but are referred to as "tumor associated" antigens. The diagnostic and therapeutic value of tumor-associated antigens that are also expressed by normal tissue results from the excess quantity of antigen expressed by tumor cells relative to normal cells and also the selectivity of the antibodies in vivo for antigen expressed by tumor cells rather than normal cells. The relative selectivity and specificity of antibodies for the antigen expressed by tumor cells is believed to result from: (1) the increased expression of the antigen on the cell membrane of the cancerous cell due to the altered and rapid metabolism of malignant growth; and (2) the increased accessibility of the antigen to antibodies administered in vivo.

To date, only a limited number of tumor-associated antigens are well characterized. Additionally, certain tumor-associated antigens useful as diagnostic or prognostic markers may not be present in all patients or during all stages and manifestations of the disease. Diagnostic discrimination and therapeutic efficacy are therefore enhanced by the ability to identify and characterize more than one tumor-associated antigen expressed by the same tumor tissue. Therefore, it is advantageous for the purposes of cancer diagnostic and therapeutics to have a set or panel of distinct antigens that are associated with specific types of human tumors. Accordingly, there exists a need for further identification and characterization of unique tumor-associated antigens.

The present invention is based upon the characterization of a novel antigen present in human tissue, including normal breast and breast carcinoma. Accordingly, the invention is directed to a distinct tumor-associated antigen, characterized by its reactivity with monoclonal antibody YBM209.

In accordance with the present invention, antibodies having specificity for the antigen defined herein and methods for the production of such antibodies are provided. Additionally, the invention is directed to the use of such antibodies for the in vitro detection and diagnosis of cancer by immunohistochemical and immunoassay methods. The invention is further directed to the use of such antibodies for the in vivo diagnosis and treatment of cancer in humans.

As indicated above, the present invention provides a novel tumor-associated antigen. In accordance with the invention, monoclonal antibody YBM209, is utilized for the characterization at least in part of this previously undescribed antigen. Monoclonal antibody YBM209 is generated by a hybrid cell line which has been deposited (April 16, 1986) with the American Type Culture Collection (ATCC) 12301 Parklawn Drive, Rockville Maryland 20852. Samples can be obtained by reference to the accession number HB9075 assigned to the cell line deposit.

The physiochemical, immunological, and imaging properties of the antigen of the present invention, and particularly, its reactivity with monoclonal antibody YBM209, permit its characterization and differentiation from other antigens present in normal human tissue and tumor tissue. Accordingly, the identifying characteristics and properties of the antigen provided by the present invention are as follows:

a) The antigen is present in human breast cell lines, specifically the T47D (ATCC deposit HTB 133) breast cell line.

b) The antigen is a membrane and cytosol component of normal breast tissue and the mucosa of breast carcinoma. Standard (ELISA) procedures (enzyme-linked immunoabsorbent binding assay) using monoclonal antibody YBM209 indicate the presence of the antigen in plasma membranes purified from human breast tissue and the mucosa of breast carcinoma. Further, 111-Indium labeled-YBM209 antibodies successfully imaged known tumor lesions in breast cancer patients in pilot studies.

c) Standard immunohistochemical procedures demonstrate the presence of the antigen in normal breast and breast carcinoma as a strong reactivity with YBM209 is shown by immunoperoxidase staining of such tissue. Reactivity is also shown with other normal tissues, including prostate, lung, liver, kidney, pituitary, skin and colon.

d) SDS - polyacrylamide gel electrophoresis (SDS-PAGE) and Western immunoblot analysis with SDS extracts of human breast cell line T47D reveal that the antigen has molecular weight component in the ranges of about 160,000 to about 175,000 44,000 to about 46,000, and 29,000 to about 35,000. Modification of the antigen by treatment with proteinase K prior to SDS-PAGE and Western immunoblot analysis destroy the antigen's reactivity with YBM209, indicating the antigen is protein in nature.

e) Isoelectric focusing of the antigen using a nonequilibrium pH gradient electrophoresis (NEPHGE) isoelectric focusing technique indicates the isoelectric point of the 29,000 to 35,000 molecular weight component is within the range of about pH 6.3 to about pH 6.9. More specifically, the isoelectric peak of the antigen is about pH 6.6.

f) The antigen is not delectable in human milk. By SDS-PAGE and Western immunoblot analysis, there is no detectable reactivity of YBM209 with human milk fat globule membranes.

g) The antigen is not associated with blood components. By fluorescence activated cell sorter analysis (FACS), there is no detectable reactivity of YBM209 with blood components, including lymphocytes, red blood cells, myeloid-components and platelets.

Summarizing the foregoing, the antigen of the present invention, which may be derived from a source selected from normal human tissue or human tumor tissue, is characterized as protein having molecular weight components within the ranges of about 160,000 to about 175,000, 44,000 to about 46,000, and 29,000 to about 35,000, and said 29,000 to about 35,000 molecular weight component having an isoelectric point within the range of about pH 6.3 to about pH 6.9 by the technique described. Further, the tumor-associated protein of the present invention is expressed by normal breast and breast carcinoma, among other normal tissues.

Of particular importance in distinguishing the antigen of the present invention from other antigens, including other tumor-associated antigens, is the specificity of monoclonal antibody YBM209 for at least one determinant of the antigen. Additionally, the specific reactivity of YBM209 for the antigen defined by the invention provides a means for isolation and purification of the antigen from other material of human origin, and ultimately, characterization of antigenic determinants. Such a purified antigen and determinants thereof are useful in the production of monoclonal and polyclonal antibodies for diagnostic and therapeutic application using techniques well known in the art. For example, murine hybridomas producing monoclonal antibodies may be obtained. Alternatively, the antigen may be used to stimulate an immune response in a rabbit, goat or other animal from whose sera polyclonal antibodies may be obtained. In addition, the antigen may be used for the characterization of antibodies of interest, e.g., monoclonal antibodies or antibodies present in human tissue, blood or other body fluids.

In accordance with the present invention, there are provided monoclonal antibodies having specificity for the novel antigens described. Further, there are provided methods for the production of these monoclonal antibodies. Preferably, such monoclonal antibodies are of the IgG class and possess an immunoreactivity with the tumor-associated protein of the present invention substantially similar to that of monoclonal antibody YBM209. Such monoclonal antibodies are useful in the detection, diagnosis and treatment of cancer in humans, particularly breast carcinoma. Further, such antibodies have application in the further isolation and purification of the antigen provided by the invention and the characterization of precise determinants.

Monoclonal antibodies as described above may be produced generally following the method of Kohler and Milstein (Nature 256, 495-497 (1975)). In accordance with the present invention, a mouse or other suitable host is immunized with the purified antigen of the invention or a membrane fraction of human tumor tissue derived from a breast carcinoma. Following immunization, the spleen cells of the immunized mouse are fused with the cells from a suitable mouse myeloma line to obtain a mixture of hybrid cell lines. The hybrid cell lines are cultured in a suitable medium and, thereafter, those hybrid cell lines producing an antibody having specific reactivity with the antigen characterized herein are selected and cloned, and the antibody produced is recovered.

The present invention suggests methods for the in vitro detection and diagnosis of cancer in humans, especially breast carcinoma. Characterization of the unique tumor-associated protein of the invention permits its detection in patient tissue samples by immunohistochemical methods and/or in patient fluid samples by in vitro immunoassay methods. A determination of the presence and/or quantity of the tumor-associated antigen of the present invention in patient specimens by immunhistochemical and/or immunoassay procedures is of diagnostic utility and may be indicative of, or correlate with, the progression of a

4

disease state.

Immunohistochemical methods for the detection of antigens in patient tissue specimens are well known to the art and need not be described in detail. Briefly, in the context of the present invention, a tissue specimen obtained from a suspected cancer patient is contacted with an antibody, preferably a monoclonal antibody, having a specificity for the tumor-associated protein characterized herein. Particularly preferred for use is monoclonal antibody YBM209. The sites at which the antibody is bound is thereafter determined by selective staining of the tissue specimen by standard immunohistochemical procedures.

Similarly, methods for the in vitro detection of antigenic substances in patient fluid samples by immunoassay procedures are well known to the art and require no repetition. For purposes of the present invention, a patient fluid sample is contacted with at least one antibody, preferably a monoclonal antibody, having specific reactivity with the tumor-associated protein of the invention and the antibody bound to sample components is determined. Qualitative and/or quantitative determinations of the presence of the antigen defined by the invention may be accomplished by competitive or non-compeitive immunoassay procedures. Monoclonal antibodies or polyclonal antibodies may be suitably utilized provided such antibodies possess the requisite specificity for the antigen provided by the present invention. Preferably, monoclonal antibody YBM209 is utilized. Additionally, the immunoassays preferred for use are two-site immunometric assays employing monoclonal antibodies which are selected to bind to non-interfering determinants of the antigen characterized herein.

The present invention further suggests methods for the in vivo diagnosis and therapy of cancer, particularly breast carcinoma. Methods for tumor localization and detection may be performed, in accordance with the present invention, by administering to a suspected cancer patient a predetermined effective amount of an antibody having specific reactivity with the tumor-associated protein of the present invention and detecting the sites of localization of the antibody by standard imaging techniques. The antibody, preferably YBM209, is administered to the patient in a pharmaceutically acceptable carrier and labeled, preferably with a radionuclide emitting gamma-radiation, so as to permit detection.

In accordance with methods permitted by the present invention for cancer therapy, a predetermined effective amount of an antibody, preferably a monoclonal antibody, having specificity for the tumor-associated antigen characterized by the invention is administered to a cancer patient. The antibody, preferably YBM209, is administered to the cancer patient is a pharmaceutically acceptable carrier and conjugated with a suitable therapeutic agent, e.g., radioisotopes, preferably emitters of beta particles, drugs, toxins or biological proteins, selected for delivery to the tumor site.

Thus, as an additional aspect of the invention, there is provided a therapeutic or diagnostic formulation which comprises a monoclonal antibody, as described, associated with a pharmaceutically- or diagnostically-acceptable carrier or diluent therefor. A pharmaceutically acceptable carrier is one which is useful in the treatment or diagnosis of a warm-blooded animal. A diagnostically-acceptable carrier is one which is useful in diagnostic testing procedures. Such carriers or diluents are completely familiar to one skilled in the art and require no further explanation.

Additionally in the context of in vivo cancer diagnosis and therapy, those skilled in the art will appreciate that antibody preparations comprising mixtures of antibodies or fragments thereof having specificity for the tumor-associated antigen of the invention may be used in certain instances to enhance the detection, localization and treatment of tumors.

Example 1

Production of Monoclonal Antibody YBM209

Lymph node lymphocytes obtained from a patient with breast carcinoma were thawed and cultured overnight in RPMI-1640 (Flow Laboratories, Alexandria, VA) containing 10% fetal bovine serum (PBS). 30 x $10^6$ lymphocytes and 30 x $10^6$ of P3/HT, (a subline of P3X63Ag8.653 (ATCC CRL 1580)) were fused with 35% polyethylene glycol-1000 in serum-free RPMI-7.5% dimethyl sulfoxide. Cells were plated out at densities of $10^5$ total cells per well in 96-well plates (Costar, Cambridge, MA) in RPMI-10% FBS - HAT ($10^{-4}$ M hypoxanthine 4 X $10^{-7}$ M aminopterin and 1.6 X $10^{-5}$ M thymidine). Cultures were maintained for two weeks in 5% $CO_2$ and thereafter supernatant was replaced with fresh HAT media once per week. Supernatant from wells with growing cultures were sampled at day 40 and tested for the presence of Ig by enzyme-linked immunoabsorbent binding assay (ELISA). Ig-producing clones were then screened by ELISA for specific reactivity with breast tumor cell lines, and subsequently with membranes and cytosols from colon tumors. The monoclonal antibody designated as YBM209 was characterized further and selected for use in the characterization of the antigen of the present invention.

5

Example 2

Antigen Characterization

Monoclonal antibody YBM209 was used to characterize the antigen of the present invention by the procedures described.

Membrane and cytosol fractions of human tissue were utilized for antigen characterization, as well a for screening the reactivity of monoclonal antibody YBM209 and were prepared as follows:

Surgical and autopsy specimens collected within ten hours after death were cut into blocks and stored at -80°C. Tissue was homogenized in four volumes of 10mM tris-HCl pH 7.5, 2mM calcium chloride, 2mM phenylmethylsulfonate (homogenization buffer) at 4°C in a Dounce homogenizer. All subsequent steps were carried out at 4°C. The homogenate was centrifuged at 100 x g for five minutes to remove nuclei and intact cells. The supernatant was removed and centrifuged at 130,000 x g for one hour. The supernatnat from this high speed centrifugation was removed and designated as the cytosol fraction. The pellet was resuspended in one volume of homogenization buffer and layered on a 40%/20% discontinuous sucrose gradient in 10mM tris-HCl pH 7.2. The gradient was centrifuged at 130,000 x g for 17 hours. Material at the 40%/20% interphase pipetted off, and diluted five-fold in homogenization buffer and centrifuged for 60 minutes at 250,000 x g. The pellet was resuspended in homogenization buffer, aliquoted and stored at -80°C.

Membrane/Cytosol ELISA

The presence of antigen reactive with monoclonal antibody YBM209 in purified membrane and cytosol fractions of normal human tissue and human tumor tissue was determined by an enzyme-linked immunoabsorbent binding assay (ELISA) as measure by absorbance at 490 nm.

To perform the membrane/cytosol ELISA, 10 $\mu$g/well of cytosol or 2$\mu$g/well of membrane, prepared as described above, were dried onto flat-bottom microtiter plates (Dynatech, Alexandria, VA) at 37°C overnight. The plates were washed three times with cold tap water. 50 $\mu$l of 20% horse serum in PBS (phosphate buffered saline, 0.15M NaCl, 20mM sodium phosphate pH 7.2) were added followed by 50 $\mu$l of hybridoma supernatant and incubated for one hour at room temperature. After washing, the wells were incubated with 100 $\mu$l of a monoclonal mouse anti-human IgM-HRP conjugate (ZSAG11, Boehringer Mannheim Biochemicals, Indianapolis, IN, 1985/86 Catalog, #952, conjugated by the method of Wilson, M.B. and Nakane, P.K., Elsevier North Holland Biomedical Press, 1978) diluted 1:1000 in 5% horse serum-PBS for one hour. Bound enzyme was detected by incubation with 100 $\mu$l of 1 $\mu$g/ml o-phenylenediamine, 0.03% of $H_2O_2$ in 0.1M citrate-phosphate buffer pH 5.0, for 30 minutes in the dark. Wells were quenched by the addition of 50 $\mu$l per well of $NH_4SO_4$. Absorbance at 490 nm was read in a Dynatech ELISA plate reader.

As shown by Table I below, YBM209 was reactive with membrane and cytosol fractions of breast carcinoma and normal tissues, indicating the presence of the antigen of the invention in breast carcinoma and normal tissue membranes and cytosol.

Biochemical Analysis of Antigen

The protein nature and molecular weight of the antigen characterized by YBM209 were determined by SDS polyacrylamide gel electrophoresis (SDS-PAGE), Western immunoblot analysis, and immunostaining. 25-50 $\mu$g of breast membrane proteins were dissolved in gel sample buffer (0.125M Tris pH 6.8, 4% SDS, 20% glycerol, 0.002% bromophenol blue), with or without 5% 2-mercaptoethanol and separated by discontinuous SDS-PAGE (Laemmli, U.K., Nature 227 680 (1970)) on a 10% or 5-15% gel. The separated proteins were electrophoretically transferred to nitrocellulose (0.1 m Schleicher and Schuell, Inc., Keene, New Hampshire) at 200mA for three hours according to the method of Towbin et al, PNAS 76 43 (1979). The nitrocellulose replicas were then incubated with 3% bovine serum albumin (BSA)-PBS for 30 minutes, after which they were incubated with hybridoma supernatant diluted 1:1 with 5% skim milk-0.001% thimerosal-0.2% anti-foam A emulsion (Sigma Chemical Co., St. Louis, MO) overnight, in sealed pouches. The nitrocellulose strips were washed with PBS-0.1% Tween for 30 minutes with five changes of buffer, and incubated with [125] I-labelled goat anti-human IgM (Tago, Burlingame, CA), 500,000 cpm/ml skim milk reagent, for two hours. After washing with PBS-0.1% Tween, the nitrocellulose strips were air-dried and exposed on x-ray film (Kodak XAR-5) for 18-72 hours with intensifying screen (Cronex DuPont Lightning Plus) at -70°C.

To further determine the nature of the antigen, membranes, prepared as described above, were subjected to the following treatments prior to SDS-PAGE and Western immunoblot analysis:

a) Periodate oxidation of antigen prior to PAGE:

1. Sample containing antigen was adjusted to 50mM $NaIO_4$ in PBS, pH 7.4 and incubated at 0°C for one hour.

2. Alternatively, sample was adjusted to 10mM $NaIO_4$ in 50mM Na acetate, pH 4.5 and incubated at 0°C for one hour.

b) Periodate oxidation of antigen after fractionation by PAGE:

Antigens were first fractionated by PAGE and subsequently immobilized on nitrocellulose paper by Western blotting. The nitrocellulose strips were incubated in 10mM $NaIO_4$, 50mM Na acetate, pH 4.5 for one hour at 4°C. Following incubation, the treated strips were incubated in 50mM $NaBH_4$ for 30 minutes at room temperature and then washed three times with PBS prior to immunostaining.

c) Neuraminidase digestion. Antigen sample was adjusted to pH 5 with 100mM HOAc and neuraminidase (Calbiochem) was added to a final concentration of 0.1 U/ml. The sample was incubated for one hour at 37°C and the digestion was stopped by the addition of SDS-PAGE sample buffer followed by heating at 100°C for five minutes.

d) Endoglycosidase F digestion. Endoglycosidase F was prepared by the method of Elder and Alexander (1982) PNAS 79:4540. Pooled fractions containing enzyme were stored in 50% glycerol at -20°C. Antigen is diluted with 0.1M phosphate buffer, pH 6.1 containing 0.05 EDTA, 1% NP-40, 0.1% SDS, 1% 2-mercaptoethanol to a final volume of 40-80 $\mu$l. The sample was boiled for two minutes, cooled and endo F was added (1:5, v:v). The sample was incubated overnight and 37°C and the reaction was stopped by addition of SDS-PAGE sample buffer followed by heating at 100°C for five minutes.

e) Proteinase K digestion. Samples containing antigen were treated with proteinase K (Sigma, Type 11) by the addition of 40 $\mu$g of enzyme/sample. Digestion was carried out at 37°C for one hour and was terminated by the addition of SDS-PAGE sample buffer followed by heating at 100°C for 15 minutes.

All reactions were stopped by adding gel sample buffer.

By SDS-PAGE and Western immunoblot analysis the molecular weight components of the antigen were determined to be within the ranges of about 160,000 to about 175,000 44,000 to about 46,000, and 29,000 to about 35,000. Chemical and enzyme modification of the antigen followed by SDS-PAGE and Western immunoblot analysis demonstrated the protein nature of the antigen. Specifically, reactivity of the antigen with YBM209 was shown to be destructible by treatment of the antigen which proteinase K as described above. The loss of reactivity of the antigen with YBM209 did not result from the alternative modifications described above.

## Isoelectric Focusing - Two-Dimensional Gel Electrophoresis

Isoelectric focusing of the 29,000 to 35,000 molecular weight component of the antigen characterized by YBM209 was determined using a non-equilibrium pH gradient electrophoresis ("NEPHGE") focusing technique on polyacrylamide gel as described below.

Two-dimensional NEPHGE gel analysis was performed using detergent extracts of T47D cells (R. Dulbecco, Salk Institute, ATCC HTB 133), essentially according to the method of O'Farrell, P.H., J.Biol. Chem. 250 4007 (1975). Nonequilibrium pH gradient electrophoresis in the first dimension with pH 3.5-pH 9.5 ampholines (LKB) was carried out at 400 volts for six hours, followed by second dimension SDS-PAGE on 5-15% gels. Electrotransfer to nitrocellulose and immunostaining analysis were then performed as described.

Isoelectric focusing of the antigen by this technique revealed its isoelectric point to be within the range of about pH 6.3 to about pH 6.9. The isoelectric point peak was at about pH 6.6.

## Analysis of Antibody Reactivity With Milk Fat Globule Membrane

To determine whether the antigen of the invention is detectable in human milk, the reactivity of YBM209 with human milk fat globule membranes was evaluated by the standard procedures described below.

Preparation of milk fat globule membranes was a follows. Human milk was centrifuged at 10,000 x g for 15 minutes. The cream, which floated to the top of the tube, was recovered and suspended in 0.01M tris-HCl buffer pH 7.5 containing 1mM $MgCl_2$ and 0.28M sucrose. This suspension was recentrifuged, and the washed cream was resuspended in 0.05M tris-HCl buffer pH 7.5. The washed milk fat globule membranes were homogenized for 10 minutes at 10,000 rpm using a Brinkman polytron. After incubation at 37°C for 20 minutes, the membranes were centrifuged at 105,000 x g for one hour at 37°. Milk fat globule membranes in the pellet were suspended in ice-cold 0.02M tris-HCl buffer pH 8.0 containing 1mM PMSF at a protein concentration of 10 mg/ml.

SDS-PAGE and Western immunoblot analysis were performed essentially as described above using 25 μg of human milk fat globule membrane. YBM209 had no detectable reactivity with the human milk fat globule membrane.

Analysis of Antibody Reactivity With Blood Components

To determine whether the antigen of the present invention is associated with blood components, the reactivity of YBM209 with blood components, including lymphocytes, red blood cells, myeloid components, and platelets was evaluated.

Freshly drawn heparinized blood was centrifuged twice at 1000 rpm for 5-8 minutes each. From this, plasma was collected and centrifuged at 2000 rpm for four to six minutes to pellet the platelets which were resuspended in an appropriate volume of plasma. The buffy layer was also collected and centrifuged twice at 1000 rpm for four to six minutes each to collect lymphocytes. 10-12 ml of 0.83% ammonium chloride-0.14% potassium bicaronate-0.05mM EDTA pH 7.3 were added to the pellet, incubated for five minutes and centrifuged again at 1000 rpm for four to six minutes. The pellet was washed once and resuspended in RPMI-10% fetal bovine serum at a concentration of 40 million cells/ml. Red blood cells were also resuspended in RPMI-10% fetal bovine serum at a concentration of 40 million cells/ml.

50 μl of human antibody supernatant were dispensed into 96-well microtiter plates, followed by 25 μl (1 million cells) of cell suspension and incubated for 30 minutes at 4°C. Cells were washed three times with PBS by centrifugation and incubated with 50 μl of goat anti-human IgM-FITC conjugate (Tago, Burlingame, CA) for 30 minutes at 4°C. Cells were again washed as before, transferred to tubes containing 0.5-1.0 ml 1% formalin and subjected to analysis on a fluorescence activated cell sorter.

The above analysis indicated no specific reactivity of YBM209 with any blood components. Therefore, the antigen of the present invention is not associated with blood components.

Human Imaging Studies

To determine the selectivity of the YBM209 antibody in vivo for the antigen of the present invention expressed by tumor cells rather than normal cells, human imaging studies on patients with recurrent breast carcinoma were performed as described below.

The feasibility and safety of using $^{111}$In-labeled monoclonal antibodies for tumor localization in human subjects has been established using anti-p97[a] murine monoclonal antibody type 96.5 (Boehringer Mannheim Biochemicals, Indianapolis, IN. 1985/86 Catalog #0202). In these studies, doses up to 20 mg of murine monoclonal antibody type 96.5 labeled with up to five mCi of $^{111}$In have been administered to over 150 patients with advance metastatic melanoma. At the optimal dose (20 mg) greater than 65% of the known lesions have been localized and detected by gamma scintigraphy.

The first patient who received the very low dose (1 mg) of the $^{111}$In-labeled antibody had five known sites of disease at the time of imaging in the ribs, sternum and skull and one subcutaneous lesion on the chest. The labeled antibody initially cleared rapidly from the circulation at one hour, although clearance rate subsequently slowed. As expected from the low dose used, none of the known lesions were imaged.

At the time of study, patient #1 had developed a right axillary lesion contiguous to her mastectomy scar. This mass was imaged by YBM209 at 24 hours and through six days after antibody infusion.

The third and forth patients had multiple bony and soft tissue lesions. The indium-labeled antibody successfully imaged known tumor lesions and in addition detected a total of four new areas of tumor involvement. Two of these new lesions were in lymph nodes, and one has since been confirmed by CT.

## TABLE 1

Monoclonal Antibody YBM209 Reactivity*
With Membranes and Cytosol Preparations

|                      | Membranes | Cytosols |
|----------------------|-----------|----------|
| Breast carcinoma 1   | 90        | 159      |
| Breast carcinoma 2   | 295       | 116      |
| Breast carcinoma 3   | 121       | 277      |
| Breast carcinoma 4   | 97        | 237      |
| Normal breast        | 203       | 132      |
| Normal benign colon  | 797       | 292      |
| Normal lung          | 273       | 324      |
| Normal liver         | 270       | 424      |
| Normal kidney        | 178       | 394      |
| Normal prostate      | 135       | 240      |
| Normal bladder       | 139       | 155      |
| Normal brain         | 22        | 80       |

*Expressed as % max = $\dfrac{\text{Test antibody}}{\text{Positive Control}}$ X 100

## Claims

Claims for the following Contracting States : BE, CH, DE, FR, GB, IT, LI, LU, NL, SE

1. An antigen derived from human tissue or a cancerous human cell line, said antigen being a protein having a molecular weight components within the ranges of about 160,000 to about 175,000, about 44,000 to about 46,000, and about 29,000 to about 35,000, said 29,000 to 35,000 molecular weight component having an isoelectric point within the range of about pH 6.3 to about pH 6.9, said antigen being further characterized by its reactivity with the monoclonal antibody YBM209 which is produced by the hybridoma cell-line ATCC HB9075.

**2.** An antigen as claimed in claim 1 in which the 29,000 to 35,000 molecular weight component of said antigen has an isoelectric point of about pH 6.6.

**3.** An antigen as claimed in claim 2 in which the reactivity of said antigen with monoclonal antibody YBM209 is destructible by treatment of said antigen with proteinase K.

**4.** An antigen as claimed in any one of claims 1 to 3 in which the antigen is a tumor-associated antigen expressed by breast carcinoma.

**5.** An antibody having reactivity against an antigen as claimed in any one of claims 1 to 4.

**6.** An antibody as claimed in claim 5 which is monoclonal antibody.

**7.** A monoclonal antibody as claimed in 6 which is a reactive to a tumor-associated protein expressed by breast carcinoma.

**8.** A monoclonal antibody as claimed in claim 7, having specific reactivity with a tumor-associated protein, said protein having molecular weight components within the ranges of about 160,000 to about 175,000 about 44,000 to about 46,000, and about 29,000 to about 35,000, the 29,000 to 35,000 molecular weight component having an isoelectric point within the range of about pH 6.3 to about pH 6.9, and a reactivity with said monoclonal antibody which is destructible by treatment of said protein with proteinase K.

**9.** Monoclonal antibody YBM209.

**10.** Hybrid cell line ATCC HB9075.

**11.** An antibody, as claimed in any one of claims 5 to 9, for use as a diagnostic or therapeutic agent.

**12.** A diagnostic or therapeutic formulation which comprises an antibody, as claimed in any one of claims 5 to 9, associated with a diagnostically- or pharmaceutically-acceptable diluent or carrier therefor.

**13.** A method for the detection and diagnosis of cancer in a patient suspected of having cancer which comprises contacting a tissue specimen or fluid sample obtained from said patient with an antibody having specific reactivity with an antigen as defined in any one of claims 1 to 4.

**Claims for the following Contracting States : AT, GR**

**1.** A process for preparing a monoclonal antibody which comprises:
(a) immunizing a host with a membrane fraction of human tumor tissue derived from a human breast carcinoma or an antigen derived from human tissue or a cancerous human cell line, said antigen being a protein having molecular weight components within the ranges of about 160,000 to about 175,000, about 44,000 to about 46,000, and about 29,000 to about 35,000, said 29,000 to 35,000 molecular weight component having an isoelectric point within the range of about pH 6.3 to about pH 6.9, and said antigen being further characterized by its reactivity with the monoclonal antibody YBM209 which is produced by the hybridoma cell-line ATCC HB9075;
(b) fusing spleen cells of said host with suitable myeloma cells to obtain a hybrid cell line;
(c) culturing said hybrid cell lines in a suitable medium and selecting and cloning those hybrid cell lines reactive to the antigen defined in (a); and
(d) recovering the monoclonal antibody produced.

**2.** A process as claimed in claim 1 in which the 29,000 to 35,000 molecular weight component of the antigen has an isoelectric point of about pH 6.6.

**3.** A process as claimed in claim 2 in which the antigen - monoclonal antibody reactivity is destructible by treatment of the antigen with proteinase K.

**4.** A process as claimed in any one of claims 1 to 3 in which the antigen is a tumor-associated antigen expressed by breast carcinoma.

5. A process as claimed in any one of claims 1 to 4 in which the hybrid cell line is ATCC HB 9075.

6. A process as claimed in any one of claims 1 to 5 in which the monoclonal antibody produced is YBM209.

7. Monoclonal antibody YBM209.

8. Hybrid cell ATCC HB 9075.

9. A diagnostic or therapeutic formulation for the detection or treatment of cancer which comprises an antibody produced by the process of any one of claims 1 to 6, associated with a diagnostically- or pharmaceutically-acceptable diluent or carrier therefor.

10. A formulation as claimed in claim 9 which comprises monoclonal antibody YBM209.

11. The use of an antigen, as defined in any one of claims 1 to 4, for the manufacture of a composition for the treatment or detection of cancer.

12. The use of a monoclonal antibody, as defined in any one of claims 1 to 7, for the manufacture of a composition for the treatment or detection of cancer.

13. A method for the detection and diagnosis of cancer in a patient suspected of having cancer which comprises contacting a tissue specimen or fluid sample obtained from said patient with an antibody as defined in any one of claims 1 to 7.

14. An antigen as defined in any one of claims 1 to 4.

15. A monoclonal antibody which is reactive to an antigen as defined in any one of claims 1 to 4.

**Claims for the following Contracting State : ES**

1. A process for preparing a monoclonal antibody which comprises:
   (a) immunizing a host with a membrane fraction of human tumor tissue derived from a human breast carcinoma or an antigen derived from human tissue or a cancerous human cell line, said antigen being a protein having molecular weight components within the ranges of about 160,000 to about 175,000, about 44,000 to about 46,000, and about 29,000 to about 35,000, said 29,000 to 35,000 molecular weight component having an isoelectric point within the range of about pH 6.3 to about pH 6.9, and said antigen being further characterized by its reactivity with the monoclonal antibody YBM209 which is produced by the hybridoma cell-line HB9075;
   (b) fusing spleen cells of said host with suitable myeloma cells to obtain a hybrid cell line;
   (c) culturing said hybrid cell lines in a suitable medium and selecting and cloning those hybrid cell lines reactive to the antigen defined in (a); and
   (d) recovering the monoclonal antibody produced.

2. A process as claimed in claim 1 in which the 29,000 to 35,000 molecular weight component of the antigen has an isoelectric point of about pH 6.6.

3. A process as claimed in claim 2 in which the antigen - monoclonal antibody reactivity is destructible by treatment of the antigen with proteinase K.

4. A process as claimed in any one of claims 1 to 3 in which the antigen is a tumor-associated antigen expressed by breast carcinoma.

5. A process as claimed in any one of claims 1 to 4 in which the hybrid cell line is ATCC HB 9075.

6. A process as claimed in any one of claims 1 to 5 in which the monoclonal antibody produced is YBM209.

**7.** Monoclonal antibody YBM209.

**8.** Hybrid cell ATCC HB 9075.

**9.** A diagnostic or therapeutic formulation for the detection or treatment of cancer which comprises an antibody produced by the process of any one of claims 1 to 6, associated with a diagnostically- or pharmaceutically-acceptable diluent or carrier therefor.

**10.** A formulation as claimed in claim 9 which comprises monoclonal antibody YBM209.

**11.** The use of an antigen, as defined in any one of claims 1 to 4, for the manufacture of a composition for the treatment or detection of cancer.

**12.** The use of a monoclonal antibody, as defined in any one of claims 1 to 7, for the manufacture of a composition for the treatment or detection of cancer.

**13.** A method for the detection and diagnosis of cancer in a patient suspected of having cancer which comprises contacting a tissue specimen or fluid sample obtained from said patient with an antibody as defined in any one of claims 1 to 7.

**Patentansprüche**
**Patentansprüche für folgende Vertragsstaaten : BE, CH, DE, FR, GB, IT, LI, LU, NL, SE**

**1.** Aus Humangewebe oder einer humanen Krebszellinie stammendes Antigen, dadurch gekennzeichnet, daß dieses Antigen ein Protein mit Molekulargewichtskomponenten in den Bereichen von etwa 160 000 bis etwa 175 000, etwa 44 000 bis etwa 46 000 und etwa 29 000 bis etwa 35 000 ist, daß die 29 000 bis 35 000er Molekulargewichtskomponente einen isoelektrischen Punkt im Bereich von etwa pH 6,3 bis etwa pH 6,9 aufweist und daß dieses Antigen eine Reaktivität mit dem monoklonalen Antikörper YBM209 aufweist, der von der Hybridomazellinie ATCC HB9075 gebildet wird.

**2.** Antigen nach Anspruch 1, wobei die 29 000 bis 35 000er Molekulargewichtskomponente des Antigens einen isoelektrischen Punkt von etwa pH 6,6 aufweist.

**3.** Antigen nach Anspruch 2, wobei die Reaktivität dieses Antigens mit dem monoklonalen Antikörper YBM209 durch die Behandlung dieses Antigens mit Proteinase K zerstört werden kann.

**4.** Antigen nach einem der Ansprüche 1 bis 3, wobei das Antigen ein tumorassoziiertes Antigen ist, das von Brustkarzinomen exprimiert wird.

**5.** Antikörper, der eine Reaktivität gegen ein Antigen nach einem der Ansprüche 1 bis 4 aufweist.

**6.** Antikörper nach Anspruch 5, der ein monoklonaler Antikörper ist.

**7.** Monoklonaler Antikörper nach Anspruch 6, der mit einem von Brustkarzinomen exprimierten tumorassoziierten Protein reagiert.

**8.** Monoklonaler Antikörper nach Anspruch 7, dadurch gekennzeichnet, daß dieser eine spezifische Reaktivität mit einem tumorassoziierten Protein besitzt, das Molekulargewichtskomponenten in den Bereichen von etwa 160 000 bis etwa 175 000, etwa 44 000 bis etwa 46 000 und etwa 29 000 bis etwa 35 000 aufweist, und dessen 29 000 bis 35 000er Molekulargewichtskomponente einen isoelektrischen Punkt im Bereich von etwa pH 6,3 bis etwa pH 6,9 aufweist, und das eine Reaktivität mit dem monoklonalen Antikörper aufweist, die durch Behandlung des Proteins mit Proteinase K zerstört werden kann.

**9.** Monoklonaler Antikörper YBM209.

**10.** Hybridzellinie ATCC HB9075.

EP 0 243 058 B1

**11.** Antikörper nach einem der Ansprüche 5 bis 9 zur Verwendung als diagnostisches oder therapeutisches Mittel.

**12.** Diagnostische oder therapeutische Formulierung, dadurch gekennzeichnet, daß sie einen Antikörper nach einem der Ansprüche 5 bis 9 zusammen mit einem diagnostisch oder pharmazeutisch annehmbaren Verdünnungsmittel oder Träger hierfür enthält.

**13.** Verfahren zur Detektion und Diagnose von Krebs in einem Patienten mit Krebsverdacht, dadurch gekennzeichnet, daß eine vom Patienten entnommene Gewebsprobe oder Flüssigkeitsprobe mit einem Antikörper zusammengebracht wird, der eine spezifische Reaktivität mit einem Antigen gemäß einem der Ansprüche 1 bis 4 aufweist.

**Patentansprüche für folgende Vertragsstaaten : AT, GR**

**1.** Verfahren zur Herstellung eines monoklonalen Antikörpers, gekennzeichnet durch:
(a) Immunisieren eines Wirts mit einer Membranfraktion von humanem Tumorgewebe, das von einem humanem Brustkarzinom stammt oder mit einem Antigen, das aus humanem Gewebe stammt oder mit einer krebsartigen Humanzellinie, wobei das Antigen ein Protein mit Molekulargewichtskomponenten in den Bereichen von etwa 160 000 bis etwa 175 000, etwa 44 000 bis etwa 46 000 und etwa 29 000 bis etwa 35 000 ist, die 29 000 bis 35 000er Molekulargewichtskomponente einen isoelektrischen Punkt im Bereich von etwa pH 6,3 bis etwa pH 6,9 aufweist, und dieses Antigen eine Reaktivität mit dem monoklonalen Antikörper YBM209 aufweist, der von der Hybridomazellinie ATCC HB9075 gebildet wird.
(b) Fusionieren von Milzzellen des Wirts mit geeigneten Myelomzellen, um eine Hybridzellinie zu erhalten.
(c) Kultivieren der Hybridzellinien in einem geeigneten Medium und Selektieren und Klonieren jener Hybridzellinien, die gegen das in (a) definierte Antigen reaktiv sind und
(d) Gewinnung des gebildeten monoklonalen Antikörpers.

**2.** Verfahren nach Anspruch 1, worin die 29 000 bis 35 000er Molekulargewichtskomponente des Antigens einen isoelektrischen Punkt bei etwa pH 6,6 aufweist.

**3.** Verfahren nach Anspruch 2, worin die Antigen-monoklonaler-Antikörper Reaktivität durch die Behandlung des Antigens mit Proteinase K zerstört werden kann.

**4.** Verfahren nach einem der Ansprüche 1 bis 3, worin das Antigen ein tumorassoziiertes Antigen ist, das von Brustkarzinomen exprimiert wird.

**5.** Verfahren nach einem der Ansprüche 1 bis 4, worin die Hybridzellinie ATCC HB 9075 ist.

**6.** Verfahren nach einem der Ansprüche 1 bis 5, worin der gebildete monoklonale Antikörper YBM209 ist.

**7.** Monoklonaler Antikörper YBM209.

**8.** Hybridzellinie ATCC HB 9075.

**9.** Diagnostische oder therapeutische Formulierung zur Detektion oder Behandlung von Krebs, dadurch gekennzeichnet, daß diese einen nach einem Verfahren der Ansprüche 1 bis 6 hergestellten Antikörper zusammen mit einem diagnostisch oder pharmazeutisch annehmbaren Verdünnungsmittel oder Träger hierfür enthält.

**10.** Formulierung nach Anspruch 9, die den monoklonalen Antikörper YBM209 enthält.

**11.** Verwendung eines in einem der Ansprüche 1 bis 4 definierten Antigens zur Herstellung einer Zusammensetzung für die Behandlung oder Detektion von Krebs.

**12.** Verwendung eines in einem der Ansprüche 1 bis 7 definierten monoklonalen Antikörpers zur Herstellung einer Zusammensetzung für die Behandlung oder Detektion von Krebs.

13

**13.** Verfahren zur Detektion und Diagnose von Krebs bei einem Patienten mit Krebsverdacht, dadurch gekennzeichnet, daß eine von diesem Patienten entnommene Gewebsprobe oder Flüssigkeitsprobe mit einem in einem der Ansprüche 1 bis 7 definierten Antikörper zusammengebracht wird.

**14.** Antigen, wie in einem der Anpsrüche 1 bis 4 definiert.

**15.** Monoklonaler Antikörper, der mit einem Antigen, wie in einem der Ansprüche 1 bis 4 definiert, reagiert.

**Patentansprüche für folgenden Vertragsstaat : ES**

**1.** Verfahren zur Herstellung eines monoklonalen Antikörpers, gekennzeichnet durch:
(a) Immunisieren eines Wirts mit einer Membranfraktion von humanem Tumorgewebe, das von einem humanem Brustkarzinom stammt oder mit einem Antigen, das aus humanem Gewebe stammt oder mit einer krebsartigen Humanzellinie, wobei das Antigen ein Protein mit Molekulargewichtskomponenten in den Bereichen von etwa 160 000 bis etwa 175 000, etwa 44 000 bis etwa 46 000 und etwa 29 000 bis etwa 35 000 ist, die 29 000 bis 35 000er Molekulargewichtskomponente einen isoelektrischen Punkt im Bereich von etwa pH 6,3 bis etwa pH 6,9 aufweist, und dieses Antigen eine Reaktivität mit dem monoklonalen Antikörper YBM209 aufweist, der von der Hybridomazellinie ATCC HB9075 gebildet wird.
(b) Fusionieren von Milzzellen des Wirts mit geeigneten Myelomzellen, um eine Hybridzellinie zu erhalten.
(c) Kultivieren der Hybridzellinien in einem geeigneten Medium und Selektieren und Klonieren jener Hybridzellinien, die gegen das in (a) definierte Antigen reaktiv sind und
(d) Gewinnung des gebildeten monoklonalen Antikörpers.

**2.** Verfahren nach Anspruch 1, worin die 29 000 bis 35 000er Molekulargewichtskomponente des Antigens einen isoelektrischen Punkt bei etwa pH 6,6 aufweist.

**3.** Verfahren nach Anspruch 2, worin die Antigen-monoklonaler-Antikörper Reaktivität durch die Behandlung des Antigens mit Proteinase K zerstört werden kann.

**4.** Verfahren nach einem der Ansprüche 1 bis 3, worin das Antigen ein tumorassoziiertes Antigen ist, das von Brustkarzinomen exprimiert wird.

**5.** Verfahren nach einem der Ansprüche 1 bis 4, worin die Hybridzellinie ATCC HB 9075 ist.

**6.** Verfahren nach einem der Ansprüche 1 bis 5, worin der gebildete monoklonale Antikörper YBM209 ist.

**7.** Monoklonaler Antikörper YBM209.

**8.** Hybridzellinie ATCC HB 9075.

**9.** Diagnostische oder therapeutische Formulierung zur Detektion oder Behandlung von Krebs, dadurch gekennzeichnet, daß diese einen nach einem Verfahren der Ansprüche 1 bis 6 hergestellten Antikörper zusammen mit einem diagnostisch oder pharmazeutisch annehmbaren Verdünnungsmittel oder Träger hierfür enthält.

**10.** Formulierung nach Anspruch 9, die den monoklonalen Antikörper YBM209 enthält.

**11.** Verwendung eines in einem der Ansprüche 1 bis 4 definierten Antigens zur Herstellung einer Zusammensetzung für die Behandlung oder Detektion von Krebs.

**12.** Verwendung eines in einem der Ansprüche 1 bis 7 definierten monoklonalen Antikörpers zur Herstellung einer Zusammensetzung für die Behandlung oder Detektion von Krebs.

**13.** Verfahren zur Detektion und Diagnose von Krebs bei einem Patienten mit Krebsverdacht, dadurch gekennzeichnet, daß eine von diesem Patienten entnommene Gewebsprobe oder Flüssigkeitsprobe mit einem in einem der Ansprüche 1 bis 7 definierten Antikörper zusammengebracht wird.

**Revendications**
**Revendications pour les Etats contractants suivants : BE, CH, DE, FR, GB, IT, LI, LU, NL, SE**

1. Antigène dérivé de tissus humains ou d'une lignée de cellules cancéreuses humaines, ledit antigène étant une protéine ayant des composantes de poids moléculaire endéans les fourchettes d'environ 160 000 à environ 175 000, d'environ 44 000 à environ 46 000 et d'environ 29 000 à environ 35 000, ladite composante de poids moléculaire de 29 000 à 35 000 ayant un point isoélectrique endéans la fourchette d'environ pH 6,3 à environ pH 6,9, ledit antigène étant, de plus, caractérisé par sa réactivité avec l'anticorps monoclonal YBM209 qui est produit par la lignée de cellules hybridomes ATCC HB9075.

2. Antigène tel que revendiqué dans la revendication 1 dans lequel la composante de poids moléculaire de 29 000 à 35 000 dudit antigène a un point isoélectrique à environ pH 6,6.

3. Antigène tel que revendiqué dans la revendication 2 dans lequel la réactivité dudit antigène avec l'anticorps monoclonal YBM209 est destructible par traitement dudit antigène par la protéinase K.

4. Antigène tel que revendiqué dans l'une quelconque des revendications 1 à 3 dans lesquelles l'antigène est un antigène associé a une tumeur exprimée par un carcinome du sein.

5. Anticorps ayant une réactivité contre l'antigène tel que revendiqué dans l'une quelconque des revendications 1 à 4.

6. Anticorps tel que revendiqué dans la revendication 5 qui est un anticorps monoclonal.

7. Anticorps monoclonal tel que revendiqué dans la revendication 6 qui est réactif vis à vis d'une protéine associée à une tumeur exprimée par un carcinome du sein.

8. Anticorps monoclonal tel que revendiqué dans la revendication 7 ayant une réactivité spécifique avec une protéine associée à une tumeur, ladite protéine ayant des composantes de poids moléculaire endéans les fourchettes d'environ 160 000 à environ 175 000, d'environ 44 000 à environ 46 000 et d'environ 29 000 à environ 35 000, la composante de poids moléculaire de 29 000 à 35 000 ayant un point isoélectrique endéans la fourchette d'environ pH 6,3 à environ pH 6,9, et une réactivité avec ledit anticorps monoclonal qui est destructible par traitement de ladite protéine par la protéinase K.

9. Anticorps monoclonal YBM209.

10. Lignée de cellule hybride ATCC HB9075.

11. Anticorps tel que revendiqué dans l'une quelconque des revendications 5 à 9 pour l'utilisation en tant qu'agent diagnostique ou thérapeutique.

12. Formulation diagnostique ou thérapeutique qui comprend un anticorps tel que revendiqué dans l'une quelconque des revendications 5 à 9 associé à un diluant ou excipient de celui-ci qui est diagnostiquement ou pharmaceutiquement acceptable.

13. Méthode de détection et de diagnose de cancer chez un patient chez lequel on suspecte un cancer qui comprend la mise en contact d'un spécimen de tissus ou d'un échantillon de fluides obtenus dudit patient avec un anticorps ayant une réactivité spécifique avec l'antigène tel que défini dans l'une quelconque des revendications 1 à 4.

**Revendications pour les Etats contractants suivants : AT, GR**

1. Procédé pour la préparation d'un anticorps monoclonal qui comprend :
   a) immunisation d'un hôte avec une fraction membranaire d'un tissu tumoral humain dérivé d'un carcinome du sein humain ou d'un antigène dérivé d'un tissu humain ou d'une lignée de cellules humaines cancéreuses, ledit antigène étant une protéine ayant des composants de poids moléculaire endéans les fourchettes d'environ 160 000 à environ 175 000, d'environ 44 000 à environ 46 000 et d'environ 29 000 à environ 35 000, ladite composante de poids moléculaire de 29 000 à 35 000 ayant un point isoélectrique endéans la fourchette d'environ pH 6,3 à environ pH 6,9, et ledit antigène étant, de plus, caractérisé par sa réactivité avec l'anticorps monoclonal YBM209 qui est produit par la lignée de cellules hybridomes ATCC HB9075 ;

b) fusion de cellules de la rate dudit hôte avec des cellules myélomiques adéquates pour obtenir une lignée de cellules hybrides ;

c) culture desdites lignées de cellules hybrides dans un milieu adéquat et sélectionnant et clonant ces lignées de cellules hybrides qui sont réactives envers l'antigène défini dans a) ; et

d) récupération des anticorps monoclonaux produits.

**2.** Procédé tel que revendiqué dans la revendication 1 dans lequel la composante de poids moléculaire de 29 000 à 35 000 de l'antigène a un point isoélectrique à environ pH 6,6.

**3.** Procédé tel que revendiqué dans la revendication 2 dans lequel la réactivité antigène-anticorps monoclonal est destructible par traitement de l'antigène par la protéinase K.

**4.** Procédé tel que revendiqué dans l'une quelconque des revendications 1 à 3 dans lequel l'antigène est un antigène associé à une tumeur exprimée par le carcinome du sein.

**5.** Procédé tel que revendiqué dans l'une quelconque des revendications 1 à 4 dans lequel la lignée de cellules hybrides est ATCC HB9075.

**6.** Procédé tel que revendiqué dans l'une quelconque des revendications 1 à 5 dans lequel l'anticorps monoclonal produit est YBM209.

**7.** Anticorps monoclonal YBM209.

**8.** Cellule hybride ATCC HB9075.

**9.** Formulation thérapeutique ou diagnostique pour la détection ou le traitement de cancers qui comprend un anticorps produit par le procédé de l'une quelconque des revendications 1 à 6, associé à un diluant ou un excipient diagnostiquement ou pharmaceutiquement acceptable de celui-ci.

**10.** Formulation telle que revendiquée dans la revendication 9 qui comprend un anticorps monoclonal YBM209.

**11.** Utilisation d'un antigène, tel que défini dans l'une quelconque des revendications 1 à 4, pour la fabrication d'une composition pour le traitement ou la détection de cancers.

**12.** Utilisation d'un anticorps monoclonal tel que défini dans l'une quelconque des revendications 1 à 7 pour la production d'une composition pour le traitement ou la détection de cancers.

**13.** Méthode pour la détection et la diagnose de cancer chez un patient chez lequel on suspecte un cancer qui comprend la mise en contact d'un spécimen de tissus ou d'un échantillon de fluides obtenus dudit patient avec un anticorps tel que défini dans l'une quelconque des revendications 1 à 7.

**14.** Antigène tel que défini dans l'une quelconque des revendications 1 à 4.

**15.** Anticorps monoclonal qui présente une réactivité envers un antigène tel que défini dans l'une quelconque des revendications 1 à 4.

**Revendication pour l'Etat contractant suivant : ES**

**1.** Procédé pour la préparation d'un anticorps monoclonal qui comprend :

a) immunisation d'un hôte avec une fraction membranaire d'un tissu tumoral humain dérivé d'un carcinome du sein humain ou d'un antigène dérivé d'un tissu humain ou d'une lignée de cellules humaines cancéreuses, ledit antigène étant une protéine ayant des composants de poids moléculaire endéans les fourchettes d'environ 160 000 à environ 175 000, d'environ 44 000 à environ 46 000

16

et d'environ 29 000 à environ 35 000, ladite composante de poids moléculaire de 29 000 à 35 000 ayant un point isoélectrique endéans la fourchette d'environ pH 6,3 à environ pH 6,9, et ledit antigène étant, de plus, caractérisé par sa réactivité avec l'anticorps monoclonal YBM209 qui est produit par la lignée de cellules hybridomes ATCC HB9075 ;

b) fusion de cellules de la rate dudit hôte avec des cellules myélomiques adéquates pour obtenir une lignée de cellules hybrides ;

c) culture desdites lignées de cellules hybrides dans un milieu adéquat et sélectionnant et clonant ces lignées de cellules hybrides qui sont réactives envers l'antigène défini dans a) ; et

d) récupération des anticorps monoclonaux produits.

**2.** Procédé tel que revendiqué dans la revendication 1 dans lequel la composante de poids moléculaire de 29 000 à 35 000 de l'antigène a un point isoélectrique à environ pH 6,6.

**3.** Procédé tel que revendiqué dans la revendication 2 dans lequel la réactivité antigène-anticorps monoclonal est destructible par traitement de l'antigène par la protéinase K.

**4.** Procédé tel que revendiqué dans l'une quelconque des revendications 1 à 3 dans lequel l'antigène est un antigène associé à une tumeur exprimée par le carcinome du sein.

**5.** Procédé tel que revendiqué dans l'une quelconque des revendications 1 à 4 dans lequel la lignée de cellules hybrides est ATCC HB9075.

**6.** Procédé tel que revendiqué dans l'une quelconque des revendications 1 à 5 dans lequel l'anticorps monoclonal produit est YBM209.

**7.** Anticorps monoclonal YBM209.

**8.** Cellule hybride ATCC HB9075.

**9.** Formulation thérapeutique ou diagnostique pour la détection ou le traitement de cancers qui comprend un anticorps produit par le procédé de l'une quelconque des revendications 1 à 6, associé à un diluant ou un excipient diagnostiquement ou pharmaceutiquement acceptable de celui-ci.

**10.** Formulation telle que revendiquée dans la revendication 9 qui comprend un anticorps monoclonal YBM209.

**11.** Utilisation d'un antigène, tel que défini dans l'une quelconque des revendications 1 à 4, pour la fabrication d'une composition pour le traitement ou la détection de cancers.

**12.** Utilisation d'un anticorps monoclonal tel que défini dans l'une quelconque des revendications 1 à 7 pour la production d'une composition pour le traitement ou la détection de cancers.

**13.** Méthode pour la détection et la diagnose de cancer chez un patient chez lequel on suspecte un cancer qui comprend la mise en contact d'un spécimen de tissus ou d'un échantillon de fluides obtenus dudit patient avec un anticorps tel que défini dans l'une quelconque des revendications 1 à 7.